# EUROPEAN PATENT APPLICATION

(11) **EP 4 197 583 A1**
(43) Date of publication of application: **21.06.2023**
(21) Application number: 20949510.0
(22) Date of filing: 12.08.2020
(51) Int. Cl.: A61M 25/09

(54) **GUIDE WIRE**

(71) Applicant: Asahi Intecc Co., Ltd., Seto-shi, Aichi 489-0071 (JP)
(72) Inventor: KONDO Daisuke, Seto-shi, Aichi 489-0071 (JP)
(74) Representative: Winter, Brandl - Partnerschaft mbB
(86) International application number: PCT/JP2020/030655
(87) International publication number: WO 2022/034648

(57) **Abstract**

Provided is improved visibility of a guide wire while improving balance of flexibility and rigidity of the guide wire. The guide wire includes a hollow shaft having a hole extended longitudinally, and a first core shaft enclosed in a hollow shaft and containing a first resin material. The first core shaft contains a radiopaque material.

## Description

### TECHNICAL FIELD

The technology disclosed herein relates to a guide wire to be inserted into a blood vessel or the like.

### BACKGROUND ART

Methods with use of a catheter are widely implemented as methods for treating or examining a constricted part or occluded part (hereinafter referred to as "lesion") in a blood vessel or the like. Typically, guide wires are used to guide a catheter to a lesion in a blood vessel or the like.

Conventionally, a guidewire is known which includes a tubular shaft having a hole extending longitudinally and a core member enclosed in the hole of the tubular shaft and formed of a polymer material (see, e.g., Patent Literature 1).

### CITATION LIST

### Patent Literature

Patent Literature 1: Japanese Unexamined Patent Application Publication No. H10-33689

### SUMMARY OF INVENTION

### TECHNICAL PROBLEM

In the conventional guide wire described above, a core member formed of a polymer material with a relatively high flexibility is enclosed in a hole of a tubular shaft, thus providing the guide wire with improved balance of flexibility and rigidity compared to a configuration employing a dense shaft having no hole. However, in the conventional guide wire, neither the tubular shaft nor the core member enclosed in the hole of the shaft contains a radiopaque material, thus providing no visibility of either the shaft or the core member under irradiation, and there is a room for improvement in visibility of a guide wire.

The specification discloses a technology that may provide a solution for the issue described above.

### SOLUTION TO PROBLEM

The technology disclosed herein can be implemented as an aspect described below.
(1) The guide wire disclosed herein includes: a hollow shaft having a hole extending longitudinally; and a first core shaft enclosed in the hole of the hollow shaft and containing a first resin material, wherein the first core shaft contains a radiopaque material. In such guide wire, the first core shaft, which contains a resin material with a relatively high flexibility, is enclosed in the hole of the hollow shaft, thus providing the guide wire with improved balance of flexibility and rigidity compared to a configuration employing a dense shaft having no hole. In the guide wire, the first core shaft also contains a radiopaque material. Thus, when the guide wire is inserted in the living body, irradiation from outside of the living body provides clear visualization of a part enclosing the first core shaft in the hollow shaft. Accordingly, the guide wire can provide improved visibility of the guide wire, and in turn, improved convenience of the guide wire. The guide wire can also contain a radiopaque material in the first core shaft, thereby providing improved visibility of a part of the guide wire. In other words, the guide wire eliminates need that a portion in a longitudinal direction of the guide wire be formed of a radiopaque material wholly from the center to the outer periphery, for the purpose of improving visibility of the part of the guide wire. This allows the guide wire to avoid losing balance of flexibility and rigidity of the guide wire for achievement of the purpose. Accordingly, the guide wire can provide improved visibility of the guide wire as well as improved balance of flexibility and rigidity of the guide wire, and in turn, improved convenience of the guide wire.
(2) The guide wire described above may have a configuration where the first core shaft contains radiopaque powder as the radiopaque material. Such guide wire can employ, as a radiopaque material, powder that is easily distributed and placed within the first core shaft, thus allow reduction of biased distribution of the radiopaque material within the first core shaft, and consequently provide effective improvement in visibility of the guide wire. In addition, adjustment of an additive amount of radiopaque powder in the first core shaft allows easy adjustment of visibility (image density) of the guide wire.
(3) The guide wire described above may have a configuration where the first core shaft contains radiopaque metal powder as the radiopaque material. Such guide wire can employ metal powder, which is relatively easily available, as a radiopaque material, thus easily providing improved visibility of the guide wire.
(4) The guide wire described above may have a configuration where the first core shaft contains fiber. Such guide wire can contain fiber in the first core shaft, thereby providing improved ductility at a position of the first core shaft within the guide wire.
(5) The guide wire described above may have a configuration where the first core shaft contains the fiber with radiopacity as the radiopaque material. Such guide wire can contain radiopaque fiber in the first core shaft, thereby providing improved ductility at a position of the first core shaft within the guide wire, as well as improved visibility of the guide wire.
(6) The guide wire described above may have a configuration where the fiber is disposed so as to extend in a longitudinal direction of the first core shaft. Such guide wire can provide effective improvement in ductility at a position of the first core shaft within the guide wire.
(7) The guide wire described above may have a configuration where in a cross-section of the first core shaft perpendicular to a longitudinal direction, the number of the fiber per unit area in a first area is larger than the number of the fiber per unit area in a second area closer to the center of the first core shaft than the first area. Such guide wire can provide improved ductility at a position of the first core shaft within the guide wire, as well as prevention of excess of an additive amount of the fiber in the first core shaft. The guide wire can also have rigidity gradually decreased from the outer periphery of the hollow shaft toward the center of the first core shaft, thus providing reduction of stress to concentrate in a radial direction of the guide wire.
(8) The guide wire described above may have a configuration where the fiber is placed only at a part in a longitudinal direction within the first core shaft. Such guide wire can provide improved ductility at a desired part within the guide wire, as well as improved fullness with the first core shaft into a hole of the hollow shaft.
(9) The guide wire described above may have a configuration further including a second core shaft enclosed at a position different from that of the first core shaft in a longitudinal direction within the hole of the hollow shaft and contains a second resin material having rigidity different from that of the first resin material. Such guide wire can adjust rigidity of each part of the guide wire in the longitudinal direction using difference in rigidity between resin materials contained in the first core shaft and the second core shaft, thereby providing more improved convenience of the guide wire.
(10) The guide wire described above may be an atherectomy guide wire to rotatably support a rotary body included in an atherectomy catheter, in a portion enclosing the first core shaft in the hollow shaft. As described above, in such guide wire, when the guide wire is inserted in the living body, irradiation from outside of the living body provides clear visualization of a part enclosing the first core shaft in the hollow shaft. This allows clear identification that a rotary body included in an atherectomy catheter is located at a part enclosing the first core shaft in the hollow shaft, thus preventing the rotary body from contacting another part of the guide wire. In addition, the guide wire itself enables identification of a blood vessel pathway, and in turn, determination whether the rotary body is capable of rotating, thus, e.g., allowing prevention of rotation of the rotary body at a highly bent site in the blood vessel, and providing improved safety.

The technology disclosed herein can be realized in various forms, e.g., in a form of a guide wire and a production method thereof.

### BRIEF DESCRIPTION OF DRAWINGS

FIG. 1 is an explanatory diagram schematically illustrating a configuration of a guide wire 100 in a first embodiment.
FIG. 2 is an explanatory diagram schematically illustrating a configuration of a guide wire 100a in a second embodiment.
FIG. 3 is an explanatory diagram schematically illustrating a configuration of a guide wire 100a in a second embodiment.
FIG. 4 is an explanatory diagram schematically illustrating a configuration of a guide wire 100b in a third embodiment.
FIG. 5 is an explanatory diagram schematically illustrating a configuration of a guide wire 100c in a fourth embodiment.
FIG. 6 is an explanatory diagram schematically illustrating a configuration of a guide wire 100d in a fifth embodiment.

### EMBODIMENTS OF THE INVENTION

### A. First Embodiment:

### A-1. Configuration of Guide Wire 100:

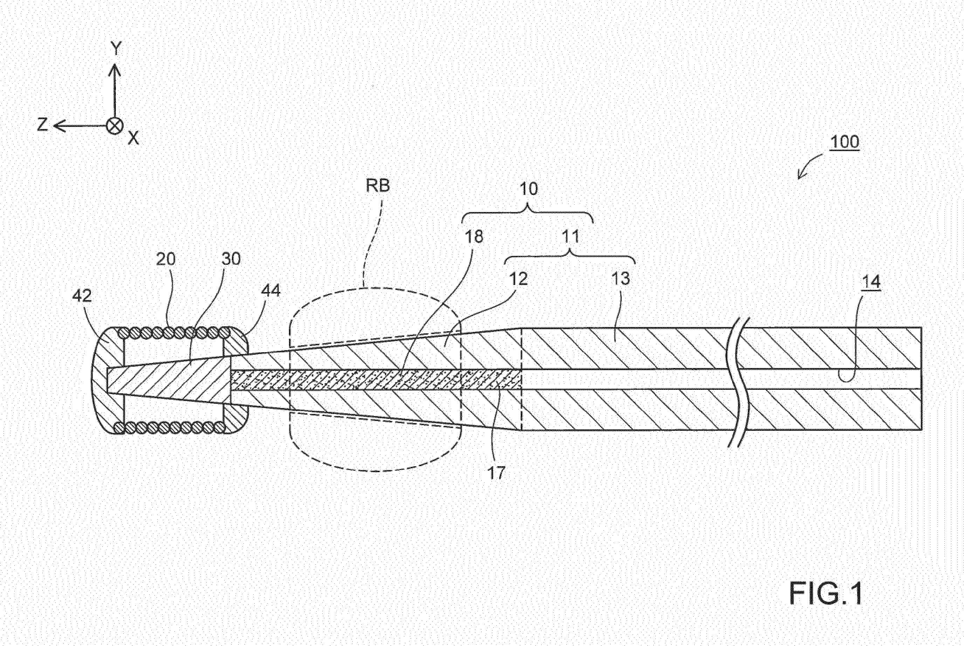

FIG. 1 is an explanatory diagram schematically illustrating a configuration of a guide wire 100 in a first embodiment. FIG. 1 illustrates a configuration of a longitudinal section (YZ-section) of the guide wire 100 in the first embodiment together with XYZ axes orthogonal to one another for specifying directions. Depiction of the guide wire 100 is partially omitted in FIG. 1. In FIG. 1, a Z-axis positive direction is toward a distal end side (far side) to be inserted into the body, and a Z-axis negative direction is toward a proximal end side (near side) to be manipulated by a technician such as a surgeon. The same applies to the other drawings. With regard to the guide wire 100 and each component thereof herein, the end on the distal end side is referred to as "distal end", the distal end and its vicinity as "distal end portion", the end on the proximal end side as "proximal end", and the proximal end and its vicinity as "proximal end portion". FIG. 1 illustrates a state where the entire of the guide wire 100 has a straight shape substantially parallel to the Z-axis direction, but the guide wire 100 is flexible enough to be curved. For convenience of explanation herein, the guide wire 100 is assumed to be in the state illustrated in FIG. 1, and the Z-axis direction is referred to as a "longitudinal direction of the guide wire 100 (and each component thereof)" or simply as a "longitudinal direction".

The guide wire 100 is a long medical device to be inserted into a blood vessel or the like, in order to guide a catheter into a lesion (constricted part or occluded part) in a blood vessel or the like. The guide wire 100 has a total length of e.g., about 1000 mm to 4000 mm.

The guide wire 100 according to the embodiment is an atherectomy guide wire for guiding an atherectomy catheter. The "atherectomy" refers to a treatment method in which a lesion (e.g., calcified lesion) is excised by e.g., using an atherectomy catheter equipped with a drill-shaped rotary body RB having micro-diamond embedded therein, and rotating the rotary body RB at a high speed (e.g., about 150,000 rpm). The rotary body RB is rotatably supported at a part (tapered portion 12) enclosing a core shaft 18 in a hollow shaft 11 described later.

The guide wire 100 includes a shaft body 10, a distal helicoid 20, a distal core material 30, a distal joint part 42, and a proximal joint part 44.

The shaft body 10 is a long member extending longitudinally. The shaft body 10 has a hollow shaft 11 and a core shaft 18.

The hollow shaft 11 of the shaft body 10 is a long member extending longitudinally. The hollow shaft 11 has a configuration that includes a large-diameter portion 13 having a substantially constant outer diameter, and a tapered portion 12 located closer to the distal end relative to the large-diameter portion 13 and having an outer diameter gradually decreasing from a boundary position with the large-diameter portion 13 toward the distal end. An outer shape of a transverse section (XY-section) at each position of the hollow shaft 11 may be any shape, such as a circular shape. An outer diameter of the large-diameter portion 13 is e.g., about 0.15 to 0.89 mm, and an outer diameter of the smallest diameter part of the tapered portion 12 is e.g., about 0.05 to 0.30 mm. A longitudinal length of the tapered portion 12 is e.g., about 30 to 500 mm.

The hollow shaft 11 has a hole 14 extending longitudinally. In the embodiment, the hole 14 is a through-hole that passes through from the distal end to the proximal end of the hollow shaft 11. A shape of a transverse section (XY-section) at each position of the hole 14 may be any shape, such as a circular shape. An inner diameter of the hole 14 is substantially constant from the distal end to the proximal end of the hollow shaft 11, e.g., about 0.05 to 0.28 mm. The inner diameter of the hole 14 need not be substantially constant from the distal end to the proximal end of the hollow shaft 11, and the inner diameters of the hole 14 at a plurality of positions in a longitudinal direction of the hollow shaft 11 may differ from one another.

As materials for forming the hollow shaft 11, known materials are used, such as metallic materials, more particularly, stainless steel (SUS302, SUS304, SUS316, etc.) and superelastic alloys such as Ni-Ti alloys. The hollow shaft 11 may be wholly formed of the same material, or may be formed of materials different from one another for portion by portion.

The core shaft 18 is a rod-shaped member enclosed in the hole 14 formed in the hollow shaft 11. In the embodiment, the core shaft 18 is enclosed in a part formed in the tapered portion 12 within the hole 14 formed in the hollow shaft 11. An outer shape of a transverse section (XY-section) at each position of the core shaft 18 may be any shape, such as a circular shape. An outer diameter of the core shaft 18 is the same as or slightly smaller than the inner diameter of the hole 14 formed in the hollow shaft 11, e.g., about 0.05 to 0.28 mm. In the embodiment, the core shaft 18 is disposed throughout a part formed in the tapered portion 12 within the hole 14 formed in the hollow shaft 11. Thus, a longitudinal length of the core shaft 18 is substantially the same as the length of the tapered portion 12 of the hollow shaft 11, e.g., about 30 to 500 mm. The longitudinal length of the core shaft 18 need not be substantially the same as the length of the tapered portion 12 of the hollow shaft 11, and may be, e.g., shorter than the length of the tapered portion 12. None is enclosed in a part formed in the large-diameter portion 13 within the hole 14 formed in the hollow shaft 11. That means, the part closer to the proximal end relative to the part enclosing the core shaft 18 within the hole 14 formed in the hollow shaft 11 is vacant. The core shaft 18 is an example of a first core shaft in the claims.

The core shaft 18 is configured to contain a resin material. More specifically, the core shaft 18 includes a rod-shape portion being formed of a resin material and having an outer shape the same as the outer shape of the core shaft 18. Examples of the resin material contained in the core shaft 18 include acrylic resins. The resin material contained in the core shaft 18 is an example of a first resin materials in the claims.

The core shaft 18 further contains radiopaque metal powder 17 as a radiopaque material (e.g., X-ray opaque material). More specifically, the metal powder 17 is placed within a rod-shaped portion formed of the resin material described above. In the embodiment, the metal powder 17 is distributed and placed substantially uniformly throughout the rod-shaped portion. Examples of the radiopaque metal powder 17 include powder of platinum, gold, silver, tin, tungsten, bismuth, rhenium, tantalum, palladium, iridium, barium, or an alloy thereof, and compounds such as bismuth oxide. Powder herein means a substance in which a ratio of the maximum dimension (length) of the substance relative to a diameter (largest diameter) of a cross section perpendicular to a direction of the maximum dimension is less than 3.0. In the embodiment, the maximum dimension of the metal powder 17 is e.g., about 0.0001 mm to 0.01 mm.

The distal core material 30 is a rod-shaped member extending longitudinally. The distal core material 30 is disposed closer to the distal end than the core shaft 18. In the embodiment, the proximal end portion of the distal core material 30 is joined to the distal end portion of the hollow shaft 11 of the shaft body 10 by e.g. brazing or welding. As materials for forming the distal core material 30, known materials are used, such as metallic materials, more particularly, stainless steel (SUS302, SUS304, SUS316, etc.) and superelastic alloys such as Ni-Ti alloys.

The distal helicoid 20 is a member formed into a spiral shape. In the embodiment, the distal helicoid 20 has a configuration including a coil body formed into a hollow cylindrical shape by densely winding one strand into a spiral shape. The distal helicoid 20 has at least a part (a substantially entire part in the embodiment) located closer to the distal end than the shaft body 10. The distal helicoid 20 is also wound so as to cover at least a part (a substantially entire part in the embodiment) of the distal core material 30.

The distal helicoid 20 is configured to contain a radiopaque material (e.g. X-ray opaque material). The radiopaque material refers to a material having radiopacity, such as radiopaque metals and radiopaque resins. As an example, the distal helicoid 20 is formed of platinum alloy.

The distal joint part 42 is a member that joins the distal end of the distal helicoid 20 with the distal end of the distal core material 30. An outer peripheral surface on the distal end of the distal joint part 42 is a smooth face (e.g. substantially hemispherical face). Meanwhile, the proximal joint part 44 is a member that joins the proximal end of the distal helicoid 20 with the distal end of the shaft body 10. As the materials forming the distal joint part 42 and the proximal joint part 44, known materials are used, such as brazing materials (aluminum alloy braze, silver braze, gold braze, etc.), metal solders (Ag-Sn alloys, Au-Sn alloys, etc.), and adhesives (epoxy-based adhesives, cyanoacrylate adhesives, etc.).

### A-2. Production Method of Guide Wire 100:

The guide wire 100 according to the embodiment can be produced as follows, for example. First, for example, a stainless steel hypotube is prepared and subjected to tapering or the like to produce the hollow shaft 11 including the tapered portion 12 and the large-diameter portion 13. In addition, a mixture of the radiopaque metal powder 17 in a resin material is filled by applying pressure from the distal end of the hollow shaft 11 into the hole 14 of the hollow shaft 11, to form the core shaft 18. In this manner, the shaft body 10 having the hollow shaft 11 and the core shaft 18 is produced.

Next, the distal core material 30 is produced, and the proximal end portion of the distal core material 30 is joined to the distal end portion of the hollow shaft 11 of the shaft body 10 by e.g., brazing or welding. Subsequently, the distal helicoid 20 is produced and joined to the distal core material 30 and the shaft body 10. Particularly, the distal joint part 42, which joins the distal helicoid 20 with the distal core material 30, and the proximal joint part 44, which joins the distal helicoid 20 with the shaft body 10, are formed by e.g., brazing. Primarily by the above-described method, the guide wire 100 according to the embodiment is produced.

### A-3. Effect of First Embodiment:

As described so far, the guide wire 100 according to the first embodiment includes the hollow shaft 11 and the core shaft 18. The hollow shaft 11 is a member having the hole 14 extending longitudinally. The core shaft 18 is enclosed in the hole 14 of the hollow shaft 11, and contains a resin material. The core shaft 18 also contains the radiopaque metal powder 17 as a radiopaque material.

As such, in the guide wire 100 according to the embodiment, the core shaft 18, which contains a resin material with relatively high flexibility, is enclosed in the hole 14 of the hollow shaft 11, thus providing improved balance of flexibility and rigidity of the guide wire 100 compared to a configuration where a dense shaft having no hole is employed.

In the guide wire 100 according to the embodiment, the core shaft 18 also contains a radiopaque material. Thus, when the guide wire 100 is inserted in the living body, irradiation from outside of the living body provides clear visualization of a part of the shaft body 10 (tapered portion 12, which is a portion enclosing the core shaft 18 in the hollow shaft 11). Accordingly, the guide wire 100 according to the embodiment can provide improved visibility of the guide wire 100, and in turn, improved convenience of the guide wire 100. For example, an operator such as a surgeon can observe a shape of the guide wire 100 curved so as to follow a blood vessel path under irradiation, thereby identifying a blood vessel pathway by the guide wire 100 itself without use of a contrast medium or the like.

The guide wire 100 according to the embodiment can also contain a radiopaque material in the core shaft 18, thereby providing improved visibility of a part of the shaft body 10. In other words, the guide wire 100 according to the embodiment eliminates need that a portion in a longitudinal direction of the shaft body 10 be formed of a radiopaque material wholly from the center to the outer periphery, for the purpose of improving visibility of the part of the shaft body 10. This allows the guide wire 100 according to the embodiment to avoid losing balance of flexibility and rigidity of the guide wire 100 for achievement of the purpose.

Accordingly, the guide wire 100 according to the embodiment can provide improved visibility of the guide wire 100 as well as improved balance of flexibility and rigidity of the guide wire 100, and in turn, improved convenience of the guide wire 100.

In the guide wire 100 according to the embodiment, the radiopaque metal powder 17 is employed as a radiopaque material to be contained in the core shaft 18. The guide wire 100 according to the embodiment can employ, as a radiopaque material, powder that is easily distributed and placed within the core shaft 18, thus allow reduction of biased distribution of the radiopaque material within the core shaft 18, and consequently provide effective improvement in visibility of the guide wire 100. In addition, adjustment of an additive amount of the radiopaque metal powder 17 in the core shaft 18 allows easy adjustment of visibility (image density) of the guide wire 100. The guide wire 100 according to the embodiment can employ the metal powder 17, which is relatively easily available, as a radiopaque material, thus easily providing improved visibility of the guide wire 100.

The guide wire 100 according to the embodiment is an atherectomy guide wire to rotatably support a rotary body RB included in an atherectomy catheter, in a portion enclosing the core shaft 18 in the hollow shaft 11 (tapered portion 12). As described above, in such guide wire 100 according to the embodiment, when the guide wire 100 is inserted in the living body, irradiation from outside of the living body provides clear visualization of a part enclosing the core shaft 18 in the hollow shaft 11. This allows clear identification that a rotary body RB included in an atherectomy catheter is located at a part enclosing the core shaft 18 in the hollow shaft 11, thus preventing the rotary body RB from contacting another part of the guide wire 100 (e.g., distal helicoid 20). In addition, the guide wire 100 itself enables identification of a blood vessel path, and in turn, determination whether the rotary body RB is capable of rotating, thus, e.g., allowing prevention of rotation of the rotary body RB at a highly bent site in a blood vessel, and providing improved safety.

### B. Second Embodiment:

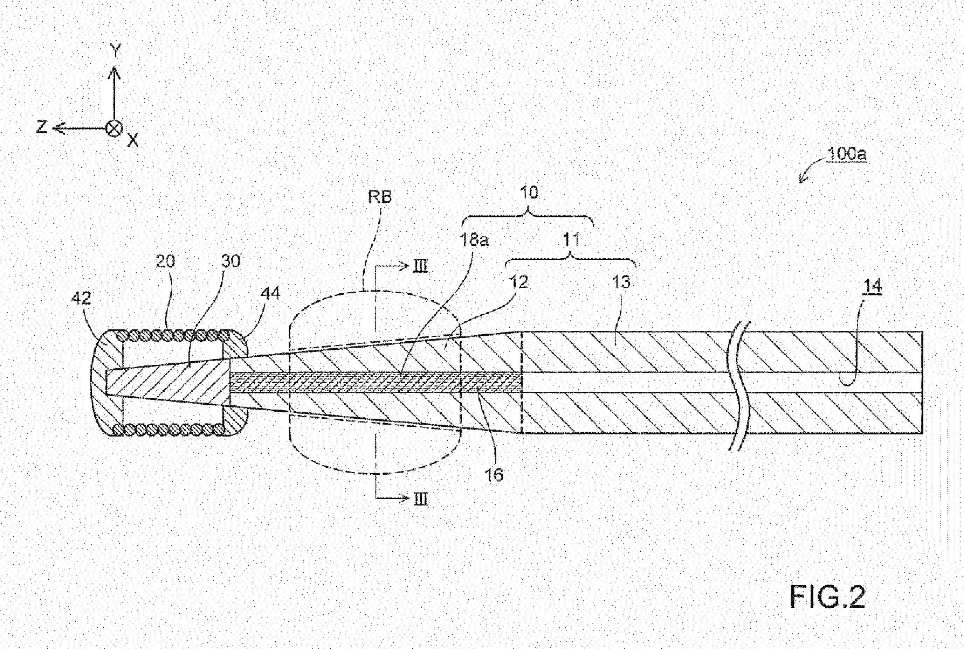

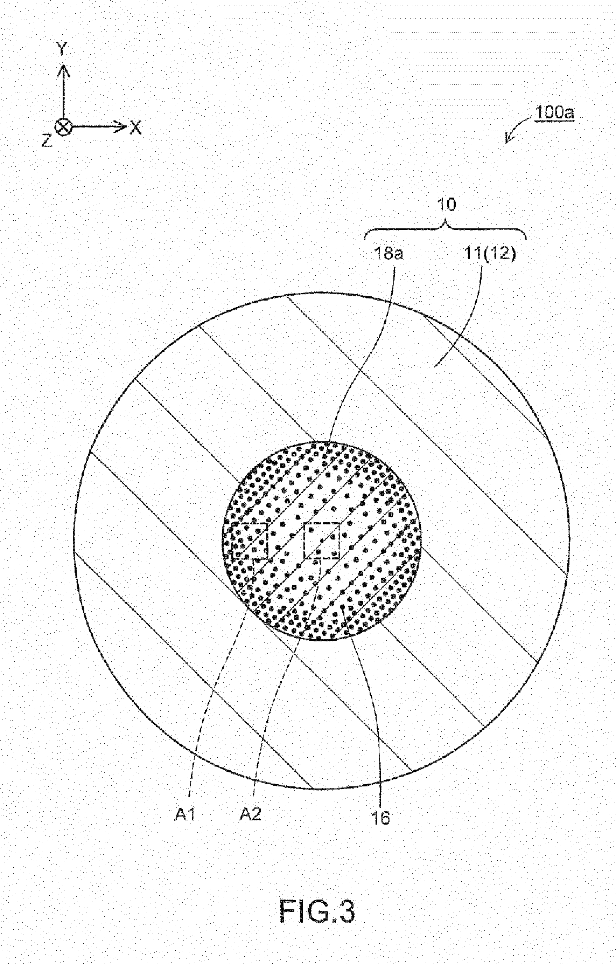

FIG. 2 and FIG. 3 are explanatory diagrams schematically illustrating a configuration of a guide wire 100a according to the second embodiment. FIG. 2 illustrates a configuration of a longitudinal section (YZ-section) of the guide wire 100a in the second embodiment, FIG. 3 illustrates a configuration of a transverse section (XY -section) of the guide wire 100a at a position III-III in FIG. 2 in an enlarged view. Hereinafter, components of the guide wire 100a according to the second embodiment that are identical to the above-described components of the guide wire 100 according to the first embodiment are provided with the same reference signs as in the first embodiment, thereby omitting the description thereof as appropriate.

The guide wire 100a according to the second embodiment has a configuration of a core shaft 18a different from that of the guide wire 100 according to the first embodiment. Particularly, in the guide wire 100a according to the second embodiment, the core shaft 18a contains a radiopaque material as well as a resin material, as in the first embodiment. However, in the guide wire 100a according to the second embodiment, the core shaft 18a contains radiopaque fiber 16 rather than the radiopaque metal powder 17 as a radiopaque material. More specifically, the fiber 16 is placed within a rod-shaped portion formed of the resin material described above, which forms the core shaft 18a. In the embodiment, the fiber 16 is distributed and placed substantially uniformly throughout the core shaft 18a in a longitudinal direction of the core shaft 18a. Meanwhile, as shown in FIG. 3, more amount of the fiber 16 is placed at a position further from the center of the core shaft 18a, in a cross section of the core shaft 18a perpendicular to the longitudinal direction. In other words, in the cross section, the number of the fiber 16 per unit area in a first area A1 is larger than the number of the fiber 16 per unit area at a second area A2, which is closer to the center of the core shaft 18a than the first area A1.

Examples of the radiopaque fiber 16 contained in the core shaft 18a include fiber of platinum, gold, silver, tin, tungsten, bismuth, rhenium, tantalum, palladium, iridium, barium, or an alloy thereof. Fiber herein means a substance in which a ratio of the maximum dimension (length) of the substance relative to a diameter (largest diameter) of a cross section perpendicular to a direction of the maximum dimension is 3.0 or more. In the embodiment, the length of the fiber 16 is e.g., about 0.003 mm to 1.0 mm, and the diameter of the fiber 16 is e.g., about 0.001 mm to 0.01 mm.

In the embodiment, each fiber 16 contained in the core shaft 18a is arranged so as to extend in a longitudinal direction of the core shaft 18a. Arrangement of each fiber 16 so as to extend in a longitudinal direction of the core shaft 18a is not limited to a configuration where an extension direction of each fiber 16 (a direction of a segment connecting one end to the other end of the fiber 16) is exactly parallel to a longitudinal direction of the core shaft 18a, but includes a configuration where an extension direction of each fiber 16 and a longitudinal direction of the core shaft 18a form an angle θ (0° ≤ θ ≤ 90°) that provides a mean value of 10° or less.

The core shaft 18a having such a configuration can be formed by, e.g., filling a mixture of the radiopaque fiber 16 in a resin material under application of pressure from the distal end of the hollow shaft 11 into the hole 14 of the hollow shaft 11, with rotating the hollow shaft 11 about a longitudinal axis.

The guide wire 100a according to the second embodiment has a configuration similar to the guide wire 100 according to the first embodiment described above, and thus exhibits an effect similar to an effect exhibited by the guide wire 100 according to the first embodiment described above.

In the guide wire 100a according to the second embodiment, the core shaft 18a contains the radiopaque fiber 16 instead of the radiopaque metal powder 17 as a radiopaque material. Therefore, the guide wire 100a according to the embodiment can contain the radiopaque fiber 16 in the core shaft 18a, thereby providing improved ductility at a position of the core shaft 18a within the guide wire 100a, as well as improved visibility of the guide wire 100a.

In the guide wire 100a according to the embodiment, the fiber 16, which is contained in the core shaft 18a, is arranged so as to extend in a longitudinal direction of the core shaft 18a, thus providing effective improvement in ductility at a position of the core shaft 18a within the guide wire 100a. Furthermore, in the guide wire 100a according to the embodiment, a cross section of the core shaft 18a perpendicular to a longitudinal direction has the number of the fiber 16 per unit area in the first area A1 larger than the number of the fiber 16 per unit area in the second area A2, which is closer to the center than the first area A1, thus providing improved ductility at a position of the core shaft 18a within the guide wire 100a, as well as prevention of excess of an additive amount of the fiber 16 in the core shaft 18a. The guide wire 100a according to the embodiment can also have rigidity gradually decreased from the outer periphery of the hollow shaft 11 toward the center of the core shaft 18, thus providing reduction of stress to concentrate in a radial direction of the guide wire 100a.

### C. Third Embodiment:

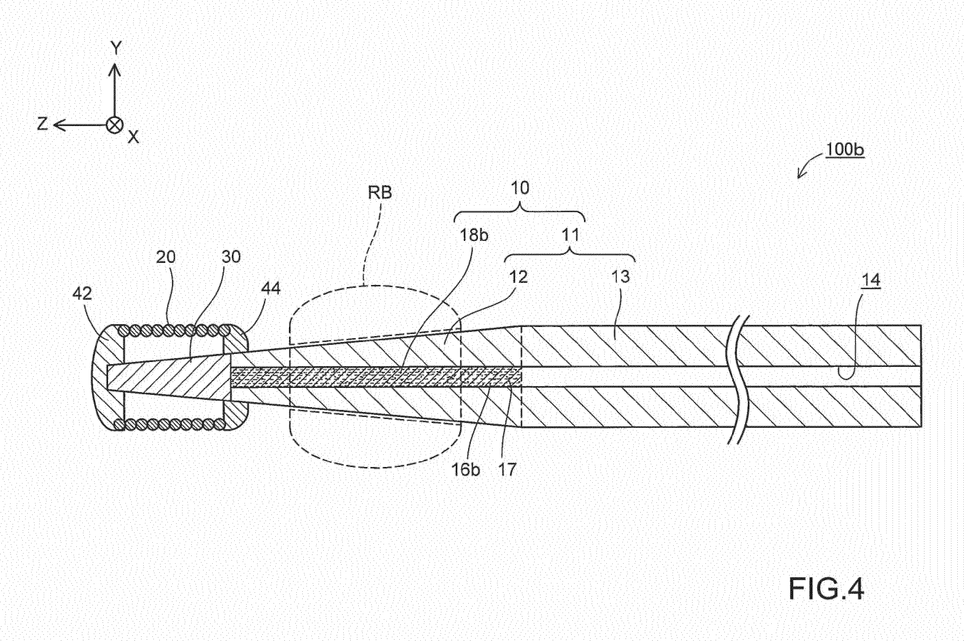

FIG. 4 is an explanatory diagram schematically illustrating a configuration of a guide wire 100b according to the third embodiment. FIG. 4 illustrates a configuration of a longitudinal section (YZ-section) of the guide wire 100b in the third embodiment. Hereinafter, components of the guide wire 100b according to the third embodiment that are identical to the above-described components of the guide wire 100 according to the first embodiment are provided with the same reference signs as in the first embodiment, thereby omitting the description thereof as appropriate.

The guide wire 100b according to the third embodiment has a configuration of a core shaft 18b different from that of the guide wire 100 according to the first embodiment. Particularly, in the guide wire 100b according to the third embodiment, the core shaft 18b contains the radiopaque metal powder 17 as a radiopaque material as well as a resin material, as in the first embodiment. However, in the guide wire 100b according to the third embodiment, the core shaft 18b further contains fiber 16b, which is formed of a material different from a radiopaque material (i.e., radiolucent). More particularly, the fiber 16b is placed within a rod-shaped portion formed of the resin material described above. Examples of the radiolucent fiber 16b contained in the core shaft 18b include radiolucent metals, resins, and the like, and carbon fiber. The configuration, position, and the like of the fiber 16b in the core shaft 18b are similar to the configuration, position, and the like of the fiber 16 in the second embodiment except that the fiber 16b is radiolucent.

The core shaft 18b having such a configuration can be formed by, e.g., filling a mixture of the radiopaque metal powder 17 and the radiolucent fiber 16b in a resin material under application of pressure from the distal end of the hollow shaft 11 into the hole 14 of the hollow shaft 11, with rotating the hollow shaft 11 about a longitudinal axis.

The guide wire 100b according to the third embodiment has a configuration similar to the guide wire 100 according to the first embodiment described above, and thus exhibits an effect similar to an effect exhibited by the guide wire 100 according to the first embodiment described above.

In the guide wire 100b according to the third embodiment, the core shaft 18b contains the radiolucent fiber 16b in addition to the radiopaque metal powder 17 as a radiopaque material. Therefore, the guide wire 100b according to the embodiment can contain the radiopaque metal powder 17 in the core shaft 18b, thereby providing improved visibility of the guide wire 100b, and can contain the radiolucent fiber 16b in the core shaft 18b, thereby providing improved ductility at a position of the core shaft 18b within the guide wire 100b. In addition, in the guide wire 100b according to the embodiment, use of the radiopaque metal powder 17 achieves improved visibility of the guide wire 100b, thus eliminates need of providing the fiber 16b with a function to improve visibility and allows increase in options of a material for the fiber 16b, and also enables employment of arrangement of the fiber 16b that provides effective improvement in ductility of the guide wire 100b at a desired position.

The guide wire 100b according to the embodiment also has a configuration of the fiber 16b similar to that of the fiber 16 in the guide wire 100a according to the second embodiment described above except for radiolucency of the fiber 16b, and thus exhibits an effect similar to an effect exhibited by the guide wire 100a according to the second embodiment described above.

### D. Fourth Embodiment:

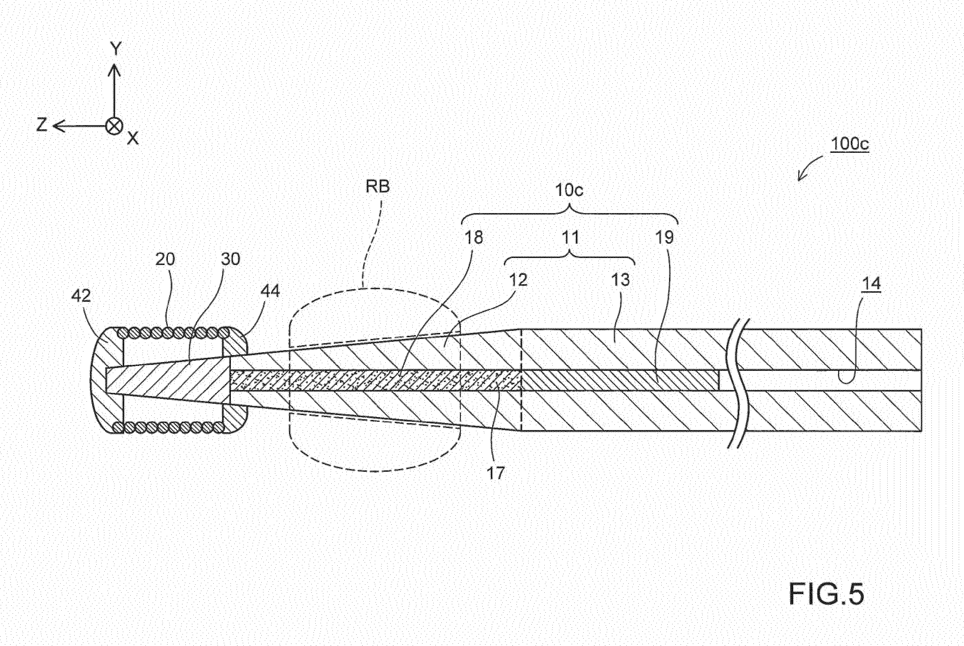

FIG. 5 is an explanatory diagram schematically illustrating a configuration of a guide wire 100c according to the fourth embodiment. FIG. 5 illustrates a configuration of a longitudinal section (YZ-section) of the guide wire 100c in the fourth embodiment. Hereinafter, components of the guide wire 100c according to the fourth embodiment that are identical to the above-described components of the guide wire 100 according to the first embodiment are provided with the same reference signs as in the first embodiment, thereby omitting the description thereof as appropriate.

The guide wire 100c according to the fourth embodiment has a configuration of a shaft body 10c different from that of the guide wire 100 according to the first embodiment. Particularly, in the guide wire 100c according to the fourth embodiment, the shaft body 10c includes a proximal core shaft 19 in addition to the hollow shaft 11 and the core shaft 18.

The proximal core shaft 19 is a rod-shaped member enclosed in the hole 14 formed in the hollow shaft 11. In the embodiment, the proximal core shaft 19 is enclosed in a part of a portion formed in the large-diameter portion 13 (a part close to the distal end) within the hole 14 formed in the hollow shaft 11. In other words, the proximal core shaft 19 is enclosed at a position different from the core shaft 18 in a longitudinal direction within the hole 14 formed in the hollow shaft 11. An outer shape of a transverse section (XY-section) at each position of the proximal core shaft 19 may be any shape, such as a circular shape. An outer diameter of the proximal core shaft 19 is the same as or slightly smaller than the inner diameter of the hole 14 formed in the hollow shaft 11, e.g., about 0.05 to 0.28 mm. A length of the proximal core shaft 19 in a longitudinal direction is e.g., about 30 to 500 mm. The proximal core shaft 19 is an example of a second core shaft in the claims.

The proximal core shaft 19 is configured to contain a resin material. More specifically, the proximal core shaft 19 is made of a resin material. A resin material contained in the proximal core shaft 19 is a resin material having rigidity different form rigidity of a resin material contained in the core shaft 18. In the embodiment, rigidity of a resin material contained in the proximal core shaft 19 is higher than rigidity of a resin material contained in the core shaft 18. For example, such a relation of rigidity is satisfied, when a resin material contained in the core shaft 18 is an acrylic resin, and when a resin material contained in the proximal core shaft 19 is an epoxy resin. Note that in the embodiment, the proximal core shaft 19 does not contain the metal powder 17, the fiber 16, or the like. A resin material contained in the proximal core shaft 19 is an example of a second resin materials in the claims.

Such a configuration of the proximal core shaft 19 can be formed by, e.g., filling a resin material under application of pressure from the distal end of the hollow shaft 11 into the hole 14 of the hollow shaft 11.

The guide wire 100c according to the fourth embodiment has a configuration similar to the guide wire 100 according to the first embodiment described above, and thus exhibits an effect similar to an effect exhibited by the guide wire 100 according to the first embodiment described above.

The guide wire 100c according to the fourth embodiment includes the proximal core shaft 19. The proximal core shaft 19 is enclosed at a position different from the core shaft 18 in a longitudinal direction within the hole 14 in the hollow shaft 11. The proximal core shaft 19 also contains a resin material having rigidity different form rigidity of a resin material contained in the core shaft 18. Therefore, the guide wire 100c according to the embodiment can adjust rigidity of each part of the guide wire 100c in the longitudinal direction using difference in rigidity between resin materials contained in the core shaft 18 and the proximal core shaft 19, thereby providing more improved convenience of the guide wire 100c.

### E. Fifth Embodiment:

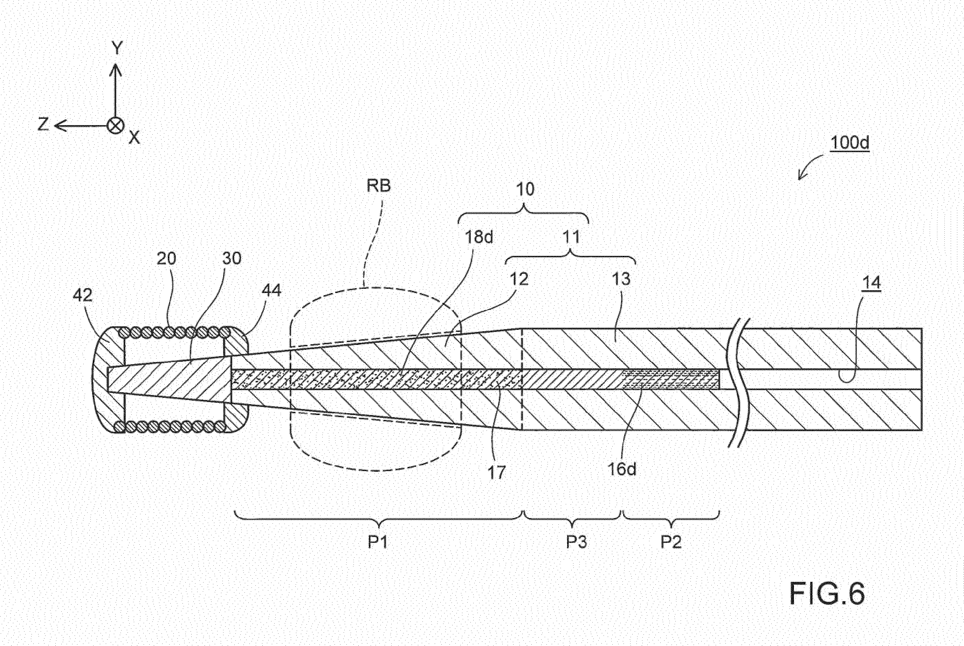

FIG. 6 is an explanatory diagram schematically illustrating a configuration of a guide wire 100d according to the fifth embodiment. FIG. 6 illustrates a configuration of a longitudinal section (YZ-section) of the guide wire 100d in the fifth embodiment. Hereinafter, components of the guide wire 100d according to the fifth embodiment that are identical to the above-described components of the guide wire 100 according to the first embodiment are provided with the same reference signs as in the first embodiment, thereby omitting the description thereof as appropriate.

The guide wire 100d according to the fifth embodiment has a configuration of a core shaft 18d different from that of the guide wire 100 according to the first embodiment. Particularly, in the guide wire 100d according to the fifth embodiment, the core shaft 18d is enclosed in an area over a part formed in the tapered portion 12 and a part close to the distal end in the large-diameter portion 13, within the hole 14 formed in the hollow shaft 11.

In the embodiment, a position of the tapered portion 12 of the hollow shaft 11 (first part P1 in FIG. 6) within the core shaft 18d contains the radiopaque metal powder 17 as a radiopaque material. Meanwhile, a position of the large-diameter portion 13 of the hollow shaft 11 (second part P2 and third part P3 in FIG. 6) within the core shaft 18d does not contain a radiopaque material. Furthermore, a part close to the proximal end (second part P2) within a position of the large-diameter portion 13 of the hollow shaft 11 in the core shaft 18d (second part P2 and third part P3) contains fiber 16d, which is formed of a material different from a radiopaque material (i.e., radiolucent). The configuration, position, and the like of the fiber 16d in the second part P2 of the core shaft 18d are similar to the configuration, position, and the like of the fiber 16 in the second embodiment except that the fiber 16d is radiolucent.

Such configuration of the core shaft 18d can be formed by, e.g., filling a mixture of the radiolucent fiber 16d in a resin material, under application of pressure from the distal end of the hollow shaft 11 into the hole 14 of the hollow shaft 11, with rotating the hollow shaft 11 about a longitudinal axis; then filling a resin material under application of pressure from the distal end of the hollow shaft 11 into the hole 14 of the hollow shaft 11; and subsequently further filing a mixture of the radiopaque metal powder 17 in a resin material under application of pressure from the distal end of the hollow shaft 11 into the hole 14 of the hollow shaft 11.

The guide wire 100d according to the fifth embodiment has a configuration similar to the guide wire 100 according to the first embodiment described above, and thus exhibits an effect similar to an effect exhibited by the guide wire 100 according to the first embodiment described above.

In the guide wire 100d according to the fifth embodiment, the fiber 16d is placed at a part in a longitudinal direction within the core shaft 18d. Thus, the guide wire 100d according to the embodiment can provide improved ductility at a desired part within the guide wire 100d as well as improved fullness with the core shaft 18d into the hole 14 of the hollow shaft 11.

The guide wire 100d according to the embodiment also has a configuration of the fiber 16d similar to that of the fiber 16 in the guide wire 100a according to the second embodiment described above except for radiolucency of the fiber 16d, and thus exhibits an effect similar to an effect exhibited by the guide wire 100a according to the second embodiment described above.

### F. Modifications:

The technology disclosed herein is not limited to the embodiments described above, and various modifications can be made within the scope that does not depart from the gist thereof. For example, the following modifications can be made.

The configuration of the guide wire 100 according to each of the above-described embodiments is merely an example and may be modified in various ways. For example, although in each of the embodiments described above, the hollow shaft 11 included in the shaft body 10 includes the large-diameter portion 13 and the tapered portion 12, the hollow shaft 11 may not have at least one of these two portions, or may have another portion in addition to the two portions. For example, the hollow shaft 11 may further have a small-diameter portion located closer to the distal end relative to the tapered portion 12 and having the same diameter as of the smallest diameter of the tapered portion 12. Alternatively, the hollow shaft 11 may further have a second tapered portion located closer to the proximal end relative to the large-diameter portion 13 and having an outer diameter gradually increasing from a boundary position with the large-diameter portion 13 toward the proximal end, and a second large-diameter portion located closer to the proximal end relative to the second tapered portion and having the same diameter as the largest diameter of the second tapered portion.

Although in each of the embodiments described above (except the fifth embodiment), the core shaft 18 is disposed throughout a portion formed in the tapered portion 12 within the hole 14 formed in the hollow shaft 11, the core shaft 18 may be disposed only at a part of a portion formed in the tapered portion 12 within the hole 14. Although in each of the embodiments described above, the core shaft 18 is enclosed in a part formed in the tapered portion 12 within the hole 14 formed in the hollow shaft 11, the core shaft 18 may be enclosed in a part formed at a portion (e.g. large-diameter portion 13) other than the tapered portion 12 of the hollow shaft 11 within the hole 14.

Although in the fifth embodiment, the hole 14 formed in the hollow shaft 11 encloses the core shaft 18 and the proximal core shaft 19, the hole 14 may enclose another core shaft containing a resin material that has rigidity different from rigidity of resin materials contained in the core shaft 18 and the proximal core shaft 19, in addition to these shafts. For example, the hole 14 formed in the hollow shaft 11 may enclose three or more core shafts containing resin materials that have rigidity different from one another. In this case, a resin material contained in each of the core shafts may be formed so as to provide rigidity increasing from the distal end to the proximal end. Moreover, when the shaft body 10 is intended to have a part with improved rigidity, a resin material with higher rigidity may be contained in a core shaft to be enclosed in the part within the hole 14 of the hollow shaft 11.

Although in each of the embodiments, the hole 14 formed in the hollow shaft 11 has a part not enclosing the core shaft 18 (or proximal core shaft 19), the entire of the hole 14 may enclose a core shaft (s) (a single core shaft, or a plurality of core shafts arranged in a longitudinal direction) that contains a resin material.

The orientation, position, and the like of the fiber 16, 16b, or 16d in the second, third, or fifth embodiments are merely an example, and a different orientation, position, and the like may be employed.

Although in each of the embodiments (except the fifth embodiment), a radiopaque material (e.g., metal powder 17 and fiber 16) is distributed and placed longitudinally through the core shaft 18, the radiopaque material may be disposed only at a part in a longitudinal direction of the core shaft 18. Although in each of the embodiments, the metal powder 17 or the fiber 16 is employed as a radiopaque material, another radiopaque material (e.g., non-metallic powder or liquid) may be employed alternatively or additionally. A resin material itself contained in the core shaft 18 may have radiopacity, and function as a radiopaque material. Although in the third and fifth embodiment, the fiber 16b and 16d are formed of a material other than a radiopaque material, the fiber 16b and 16d may be formed of a radiopaque material.

Although in each of the embodiments, the hole 14 of the hollow shaft 11 passes through from the distal end to the proximal end of the hollow shaft 11, the hole 14 of the hollow shaft 11 may be formed only at a part in a longitudinal direction of the hollow shaft 11. For example, the hole 14 of the hollow shaft 11 may open at the distal end of the hollow shaft 11, and may not open at the proximal end of the hollow shaft 11.

Although in each of the embodiments, the distal core material 30 and the distal helicoid 20 are disposed close to the distal end of the shaft body 10, at least one of the distal core material 30 and the distal helicoid 20 may be omitted.

The material of each member in each of the embodiments described above is merely an example and may be modified in various ways. The production method of the guide wire 100 according to each of the embodiments described above is also merely an example and may be modified in various ways. For example, although in each of the embodiments described above, the core shaft 18 is formed by filling a resin material into the holes 14 of the hollow shaft 11 under pressure, the core shaft 18 may be formed by filling a two-component resin material into the holes 14 of the hollow shaft 11 and allowing it to cure.

Although each of the embodiments described above has been explained with reference to the guide wire 100 for atherectomy, the technology disclosed herein can be similarly applied to guide wires for other uses.

### DESCRIPTION OF REFERENCE NUMERALS

10 shaft body
11 hollow shaft
12 tapered portion
13 large-diameter portion
14 hole
16 fiber
17 metal powder
18 core shaft
19 proximal core shaft
20 distal helicoid
30 distal core material
42 distal joint part
44 proximal joint part
100 guide wire
A1 first area
A2 second area
P1 first part
P2 second part
P3 third part
RB rotary body

## Claims

1. A guide wire comprising:
a hollow shaft having a hole extended longitudinally, and
a first core shaft enclosed in the hole of the hollow shaft and including a first resin material,
wherein the first core shaft includes a radiopaque material.

2. The guide wire according to claim 1,
wherein the first core shaft includes radiopaque powder as the radiopaque material.

3. The guide wire according to claim 2,
wherein the first core shaft includes radiopaque metal powder as the radiopaque material.

4. The guide wire according to any one of claim 1 to claim 3,
wherein the first core shaft includes fiber.

5. The guide wire according to claim 4,
wherein the first core shaft includes the fiber with radiopacity as the radiopaque material.

6. The guide wire according to claim 4 or claim 5,
wherein the fiber is arranged so as to extend in a longitudinal direction of the first core shaft.

7. The guide wire according to any one of claim 4 to claim 6,
wherein in a cross section of the first core shaft perpendicular to a longitudinal direction, the number of the fibers per unit area in a first area is larger than the number of the fiber per unit area in a second area closer to the center of the first core shaft than the first area.

8. The guide wire according to any one of claim 4 to claim 7,
wherein the fiber is placed only at a part in a longitudinal direction within the first core shaft.

9. The guide wire according to any one of claim 1 to claim 8, further comprising:
a second core shaft enclosed at a position different from the first core shaft in a longitudinal direction within the hole of the hollow shaft, and including a second resin material having rigidity different from rigidity of the first resin material.

10. The guide wire according to any one of claim 1 to claim 9,
wherein the guide wire is an atherectomy guide wire to rotatably support a rotary body included in an atherectomy catheter, in a portion enclosing the first core shaft in the hollow shaft.
